Europäisches Patentamt

European Patent Office

Office européen des brevets

(11) Publication number: **0 222 473**
**A1**

(12) **EUROPEAN PATENT APPLICATION**

(21) Application number: **86306498.6**

(22) Date of filing: **21.08.86**

(51) Int. Cl.⁴: **A 61 N 1/04**
**A 61 B 5/04**

(30) Priority: **27.08.85 JP 187597/85**

(43) Date of publication of application:
**20.05.87 Bulletin 87/21**

(84) Designated Contracting States:
**DE FR GB**

(71) Applicant: **KUREHA KAGAKU KOGYO KABUSHIKI KAISHA**
**9-11 Horidome-cho 1-chome Nihonbashi Chuo-ku Tokyo(JP)**

(72) Inventor: **Shigeta, Masatomo**
**160-1, Harada Nishikimachi**
**Iwaki-shi Fukushima-ken(JP)**

(72) Inventor: **Abe, Hikonori**
**78-21 Hananoi Nishikimachi**
**Iwaki-shi Fukushima-ken(JP)**

(72) Inventor: **Nishiyama, Shinichi**
**255-2, Takijirisadanota Izumi-machi**
**Iwaki-shi Fukushima-ken(JP)**

(74) Representative: **Marlow, Nicholas Simon et al,**
**Reddie & Grose 16, Theobalds Road**
**London WC1X 8PL(GB)**

(54) **Electrode for a living body.**

(57) An electrode for a living body comprises a main body (2) consisting of an electroconductive rubber containing fine particles or fibers of carbon and a lead (3) of flexible graphite, the whole of the electrode being substantially transparent to X-rays so that the electrode is not a hindrance to X-ray inspection.

FIG.3B

EP 0 222 473 A1

This invention relates to an electrode for a living body which is suitable for a defibrillator, an electrocardiograph, or the like.

Description of the Prior Art:

In the process of a cardiac catheterization, a catheter is inserted into a blood vessel of a patient to inject a contrast media for X-ray inspection. There is, however, the case that the patient's heart is convulsed for more than 15 to 30 seconds due to an excitation in the middle of the inspection. If the convulsion of the heart continues for a long time, it causes damage to brain cells through interruptions of blood flow. Accordingly, when such a convulsion of the heart arises, in general, a high-voltage shock of 3000 to 5000 volts is given to the heart with a defibrillator.

Such a conventional defibrillator has generally a box-shaped body provided with a handle at the upper surface of the body. The defibrillator is provided with electrodes at the lower surface of the body. The electrodes are to be brought into contact with a patient's body at the portion over his heart to give an electric shock to the heart.

Such a conventional defibrillator is, however, very troublesome because it must be brought out to be used whenever the patient's heart is convulsed. Especially, there is the possibility that it is not in time for the case of emergency.

Besides, on several occasions, the electrodes of an electrocardiograph must be attached to the surface of a patient's body for getting electrocardiograms at the time of an X-ray inspection. But, because the electrodes of such an electrocardiograph are conventionally made of metal and they are not transparent in regard to X rays, they are hindrances to X-ray photographing.

Accordingly, it is an object of the present invention to provide an electrode for a living body which is substantially transparent to X rays so as not to hinder any X-ray inspection.

Advantageously, the electrode can serve both as electrodes of an electrocardiograph and a defibrillator.

According to the invention there is provided an electrode for a living body comprising a main body which consists of an electroconductive rubber containing fine particles of fibers of carbon, at least the surface of the electrode to contact with a living body being flexible and the whole of the electrode being substantially transparent to X-rays.

The invention will be further described by way of example with reference to the drawings, in which:

Fig.1 is a cross-sectional view showing an electrode of a defibrillator according to an embodiment of the invention;

Figs.2A and 2B are a side view and a plan view showing a manner of use of the defibrillator; and

Figs.3A and 3B are perspective views of electrodes with lead portions.

Hereinafter, embodiments in which the present invention is applied to the electrodes of a defibrillator will be described with reference to attached drawings.

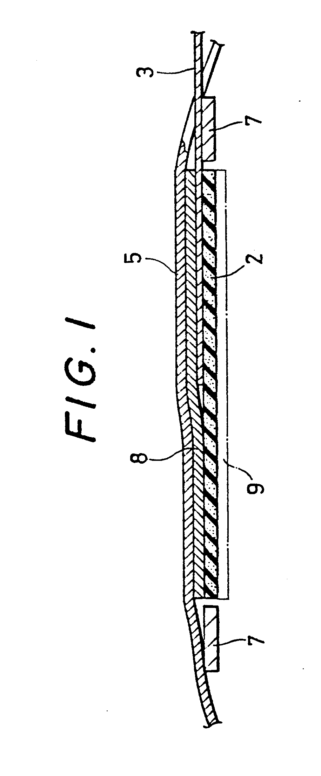

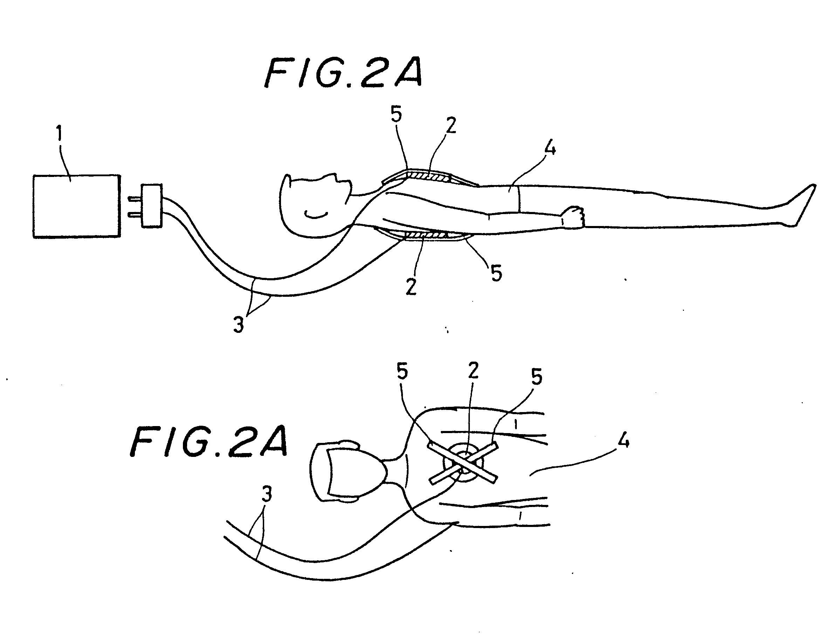

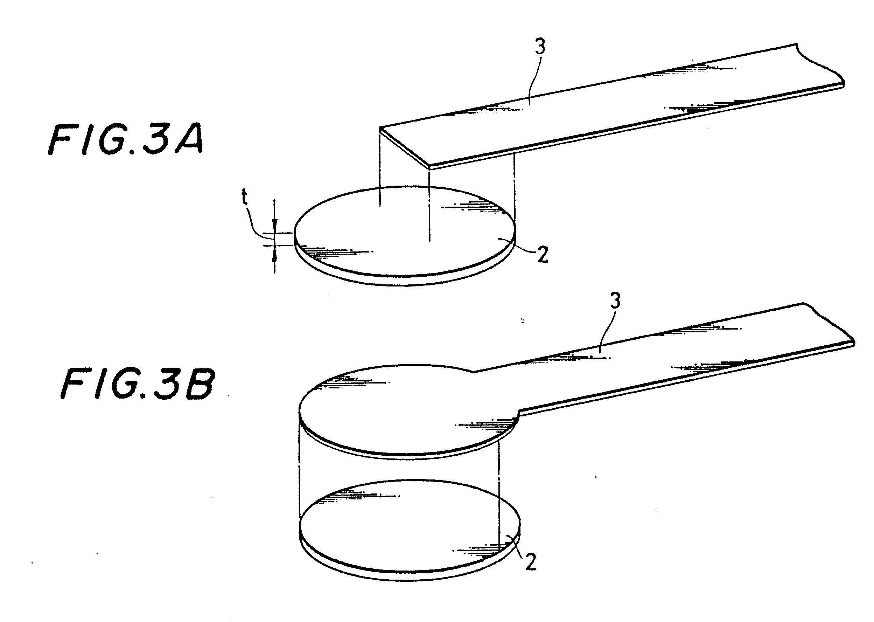

Referring first to Figs.2A and 2B, a defibrillator comprises a high-voltage source 1, a pair of electrode bodies (hereinafter, each merely called " electrode ") 2, and leads 3 for connecting the electrodes to the high-voltage source. Each electrode 2 consists of a disk-like electroconductive rubber (see Figs.3A and 3B) having the area of about 50cm² and the thickness of about 0.4 mm. The electrodes 2 are attached to a patient's body 4 from both of the breast side and the back side of his heart, respectively. As shown in Figs.1, 2A and 2B, each electrode 2 is directly attached to the patient's skin with a proper adhesive and fixed with adhesive tapes or bands 5. As the present case, when the electrodes serve as that of a defibrillator, they must be fixed in an especially firm manner because they are apt to come off from the patient's body due to a high-voltage shock.

Referring to Fig.1, a ring-shaped sponge 7 is arranged around the electrode 2. A protective layer 8 is disposed on the upper surface of the electrode 2. As shown by imaginary lines in Fig.1, a buffer 9 such as a sponge containing physiological sodium chloride solution may be interposed between the electrode 2 and a living body in order to improve the affinity with and the followability to the living body and the absorbability of a high-voltage shock.

Each electrode 2 is made of an electroconductive rubber containing fine particles or fibers of carbon. The matrix of such an electroconductive rubber may be natural rubber, Neoprene (trade name by Du Pont), butadiene rubber, silicone rubber, etc.

The carbon materials to be mixed in the above matrix may be powder of carbon black or fibers of carbon having the length of less than 3 mm. The content of the carbon materials preferably falls within the range of 30 to 90 wt. %, more preferably within the range of 40 to 80 wt.%. If the content is too little, the electrical conductivity required can not be obtained, while, if the content is too much, the elasticity or flexibility of the electrode decreases.

The thickness t of the electrode 2 as shown in Fig.3A preferably falls within the range of 0.1 to 2 mm, more preferably within the range of 0.2 to 1 mm. If the thickness t is less than 0.1 mm, the mechanical strength of the electrode decreases, while, if the thickness t is more than 2 mm, the flexibility of the electrode decreases.

In addition, particularly in the case of the electrodes of a defibrillator, since a high voltage (3000 to 5000 volts) is applied to and a large current flows through them, the electrical resistance of each electrode is preferably less than 1. 0 ohm. If the resistance is too large, the electrode may cause a burn on a living body due to the generation of heat.

Each lead 3 of the defibrillator is made of a flexible sheet of graphite such as Grafoil (trade name by Union Carbide Corp). Figs.3A and 3B show examples that the lead 3 is made of Grafoil. In the example shown in Fig.3B, the contact area between the electrode 2 and the lead 3 is made larger to increase the adhesive strength and decrease the contact resistance.

Grafoil can be easily formed into any shape by punching or the like so it is superior in mass productivity. In addition, Grafoil can make the contact resistance between the lead and the electrode decrease to a value which may be disregarded.

The electrode 2 and the lead 3 can be bonded to each other with an electroconductive adhesive that powder of carbon black is mixed in a thermosetting resin. The thermosetting resin usable is, for example, phenolic resin, epoxy resin, etc. The content of carbon black is preferably 30 to 90 wt. %. If the content of carbon black is too little, the electrical cnductivity required can not be obtained, while, if the content is too much, the adhesive strength decreases.

Also an electroconductive adhesive that powder of carbon black is mixed in a rubber adhesive can be used. In that case, either of a natural rubber adhesive or a synthetic rubber adhesive can be used. The content of carbon black is preferably 30 to 90 wt.% from the same reason as described above. An electroconductive adhesive of this type can make the connecting portion between the electrode and the lead flexible though the adhesive strength may decrease.

In the above-mentioned configuration, both of the electrodes 2 and the leads 3 are substantially transparent in regard to X rays. Therefore, they are not hindrances to X-ray photographing. As the result, the electrode can be attached to a patient's body through an X-ray inspection.

In the present invention, also a lead made of carbon fibers can be used. In that case, however, the connecting manner of the lead to an electrode may become difficult. In addition, there are disadvantages that the electrical resistance increases and the lead becomes bulky. The above-mentioned lead 3 of Grafoil can solve all of those problems.

In the above-mentioned embodiments, the present invention is applied to the electrodes of a defibrillator. The present invetion, however, can be applied also to the electrodes of an electrocardiograph. In addition, an electrode of the present invention can serve both as electrodes of a defibrillater and an electrocardiograph. Furthermore, the present invention can be applied to various electrodes such as that of an electroencephalograph and so on.

As described above, in the present invention, the main body of an electrode consists of an electroconductive rubber containing fine particles or fibers of carbon and the whole of the electrode is substantially transparent in regard to X rays. Threrfore, the electrode is not a hindrance to X-ray photographing. As the result, it can be applied to the electrodes of an electrocardiograph or a defibrillater attached to a living body through an X-ray inspection. In addition, in the present invention, since the surface of an electrode to contact with a living body is made flexible, the followability to the living body is superior so the electrode is hard to come off from the living body even when it is used as an electrode of a defibrillator.

## CLAIMS

1.  An electrode for a living body comprising a main body (2) which consists of an electroconductive rubber containing fine particles of fibers of carbon, at least the surface of the electrode to contact with a living body being flexible and the whole of the electrode being substantially transparent to X-rays.

2.  An electrode according to claim 1, in which the matrix of the electroconductive rubber is natural rubber, Neoprene, butadiene rubber or silicone rubber.

3.  An electrode according to claim 1 or 2, in which the carbon contained in the electroconductive rubber is powder of carbon black or fibers of carbon having the length of less than 3 mm.

4.  An electrode according to any preceding claim, in which the carbon content is within the range of 30 to 90 wt.%.

5.  An electrode according to any preceding claim, in which a lead (3) for connecting the main body (2) of the electrode to an external electric source comprises a flexible sheet of graphite.

6.  An electrode according to claim 5, in which the lead (3) is bonded to the main body (2) of the electrode with an electroconductive adhesive comprising fine particles of carbon in a thermosetting resin or a rubber adhesive.

# FIG. 1

FIG.2A

FIG.2A

FIG.3A

FIG.3B

# 0222473

| DOCUMENTS CONSIDERED TO BE RELEVANT | | | EP 86306498.6 | |
|---|---|---|---|---|
| Category | Citation of document with indication. where appropriate, of relevant passages | Relevant to claim | CLASSIFICATION OF THE APPLICATION (Int. Cl 4) | |
| Y | US - A - 3 566 860 (LUCAS H. MOE) | 1 | A 61 N 1/04 | |
| A | * Abstract; column 2, lines 23-27; fig. 1 * | 2-4 | A 61 B 5/04 | |
| | -- | | | |
| Y | US - A - 4 442 315 (SEGAWA) | 1 | | |
| | * Abstract; fig. 1-3 * | | | |
| | -- | | | |
| X,P | EP - A1 - 0 174 165 (RAYCHEM) | 1-4 | | |
| | * Abstract; page 9, lines 1-9; page 13, lines 18-29; page 18, claim 2 * | | | |
| | -- | | | |
| Y | US - A - 3 606 881 (WOODSON) | 1 | | |
| A | * Abstract; column 2, lines 26-31; fig. 1-5 * | 2-4 | | |
| | -- | | TECHNICAL FIELDS SEARCHED (Int Cl 4) | |
| Y | US - A - 4 370 984 (CARTMELL) | 1 | A 61 N | |
| | * Abstract; fig. 1-5 * | | A 61 B | |
| | -- | | | |
| A | DE - A - 2 209 430 (WOHLMUTH) | 1-4 | | |
| | * Page 1, line 1 - page 2, line 5; page 7, claim 1 * | | | |
| | -- | | | |
| Y | EP - A1 - 0 097 436 (MINNESOTA MINING) | 1 | | |
| A | * Abstract; page 4, lines 20-26 * | 2-4 | | |
| | -- | | | |

The present search report has been drawn up for all claims

| Place of search | Date of completion of the search | Examiner |
|---|---|---|
| VIENNA | 30-01-1987 | NEGWER |

0222473

European Patent
Office

EUROPEAN SEARCH REPORT

Application number

-2-

EP 86306498.6

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| Y | US - A - 4 257 424 (CARTMELL) <br> * Abstract; fig. 1-5 * <br> -- | 1 | A 61 N 1/04 <br> A 61 B 5/04 |
| A | DE - A - 2 260 564 (SAKAI) <br> * Page 7, line 32 - page 8, line 4; fig. 3,4 * <br> ---- | 1,5,6 | |

|  | TECHNICAL FIELDS SEARCHED (Int. Cl 4) |
|---|---|
| | A 61 N <br> A 61 B |

The present search report has been drawn up for all claims

| Place of search | Date of completion of the search | Examiner |
|---|---|---|
| VIENNA | 30-01-1987 | NEGWER |